Europäisches Patentamt

**European Patent Office**

Office européen des brevets

(11) Publication number : **0 475 584 A2**

# (12) EUROPEAN PATENT APPLICATION

(21) Application number : **91307305.2**

(22) Date of filing : **08.08.91**

(51) Int. Cl.$^5$ : **C12N 15/29, C12N 15/82, C07K 15/00**

(30) Priority : **12.09.90 US 581821**

(43) Date of publication of application :
**18.03.92 Bulletin 92/12**

(84) Designated Contracting States :
**AT BE CH DE DK ES FR GB GR IT LI LU NL SE**

(71) Applicant : **PIONEER HI-BRED INTERNATIONAL, INC.**
**700 Capital Square 400 Locust Street**
**Des Moines Iowa 50309 (US)**

(72) Inventor : **Bowen, Ben**
**3008 36th Street**
**Des Moines, Polk County, Iowa 50310 (US)**
Inventor : **Roth, Brad.**
**210 N.W. 3rd Place**
**Grimes, Polk County, Iowa 50111 (US)**

(74) Representative : **Goldin, Douglas Michael et al**
**J.A. KEMP & CO. 14, South Square Gray's Inn**
**London WC1R 5EU (GB)**

(54) Inactivation of gene transcription in plants using altered transcriptional activators.

(57) Altered transcriptional activators can bind to genes or other components of the transcriptional machinery of the cell without activating transcription of the genes. Synthetic DNA sequences which code for these modified regulatory compounds can be introduced into plant cells to competitively inhibit expression of one or more target genes.

EP 0 475 584 A2

## Technical Field

This invention relates to the use of artificially modified transcriptional activators to inactivate transcription of genes in plants.

## Background of the Invention

In the transformation of plant cells to alter the structure and/or function of whole plants, it is sometimes desirable to block the expression of a gene. For example, blocking a gene whose expression is necessary for pollen or anther formation produces male sterility. As another example, blocking the the gene which codes for the enzyme which catalyzes the conversion of sugars to starch can be used to produce sweet corn which not only has high levels of sweetness but also retains its sweetness longer after harvesting. As yet another example, blocking the gene which codes for diglycerol acyl transferase can be used to produce plants having storage lipids which consist principally of mono- and diglycerides. Still another example is the blocking of genes which code for the enzymes responsible for fatty acid elongation and/or unsaturation to produce plants having storage lipids which are rich in palmitates or in saturates.

It is well known that genes are expressed through a multi-step pathway, one step of which is transcription of the double-stranded RNA sequence to a corresponding single-stranded messenger RNA (mRNA) sequence. During transcription, the sense strand of a gene separates from its antisense partner. Enzymes then assemble an RNA molecule that complements the sequence on the antisense DNA strand. This messenger RNA eventually migrates to cell structures called ribosomes, which read the encoded information and string together the appropriate amino acids to form the encoded proteins. Blocking DNA transcription interrupts this process at its origin and effectively blocks expression of a gene. Thus, for example, if it is desired to create a plant in which a particular biochemical pathway is blocked, one means of doing so would be to prevent formation of an essential enzyme in the pathway. Since enzymes are protein molecules which are formed as a result of expression of a gene, it is possible to block the entire pathway by blocking transcription of the gene which codes for an enzyme or by blocking translation of the RNA transcript.

In the past, it has been found that expression of a gene can be blocked by the use of antisense technology. In the antisense system, antisense RNA or single-stranded DNA which is complementary to sense RNA is produced which hybridizes with sense RNA, rendering it incapable of serving its usual function. For example, replication of some plasmids requires RNA primers. In the duplexed state, the RNA primer cannot initiate DNA replication because it cannot pair with the replication origin of the plasmid. The utility of antisense RNA goes beyond regulation of DNA replication; it also extends to regulation of transcription and translation. During transcription, messenger RNA encoding the gene product is generated from the structural gene. When the cell also transcribes antisense RNA from the sense DNA strand of the gene, the antisense RNA is able to bind specifically with the sense messenger RNA and prevent the ribosome from translating the encoded information into a protein. An example of the use of this technology can be found in U.S. Patent 4,943,674, issued July 24, 1990 to Houck et al. and assigned to Calgene, Inc. However, antisense has limited effectiveness because it only reduces the amount of a particular RNA sequence available for translation in the cell, rather than blocking its action entirely. Accordingly, there exists a continuing need for new means of more effectively blocking the expression of genes.

It is also desirable in some instances to block expression of entire sets of coordinately regulated genes. In such instances, use of antisense technology becomes prohibitively complex, because an antisense molecule is highly specific -- it only binds to one complementary RNA sequence and therefore it only affects expression of a single gene. In order to completely block expression of sets of coordinately regulated genes, it would be necessary to generate antisense molecules for each gene in the set, or for the regulatory protein for the set. It would be desirable to block expression in such cases with a single gene modification.

Transcription of eukaryotic genes is influenced by various regulatory elements, including transcriptional regulatory proteins which bind to DNA in a sequence-specific manner. A large number of these proteins has been identified. The manner in which the sequence specific binding of these proteins is implemented can be divided into a number of categories. As described in the recent review article by Johnson et al., Annu. Rev. Biochem., 58:799-839 (1989) the disclosures of which are well known to the art, these include:

- helix-turn-helix recognition and binding, in which two alpha helical segments are separated by a relatively sharp beta turn;
- zinc finger recognition and binding, in which repetitive sequences in the protein sequester zinc ions to form loops which interact with the DNA;
- leucine zipper recognition and binding, in which a heptad array of leucine residues provides an interlocking helical structure for dimerization. This dimerization orients basic regions toward the N-terminal end of the dimer from the "zipper" for interaction with a specific DNA sequence;
- helix-loop-helix recognition and binding, in which a basic region is oriented similar to the leucine zipper mechanism but in which dimerization occurs via interaction of two alpha helical structures separated by a loop; and
– unclassified binding systems.

Likewise, activation domains can be classed as rich in acidic residues, glutamine residues, or proline residues. This is reviewed at length by Mitchell et al., Science, 45:371-378B (1989).

Dominant trans-acting inhibitors have been identified for genes encoding transcriptional activators in yeast and mammalian cells. The action of these inhibitors has been demonstrated using heterologous reporter genes in transient assays.

In plants, a naturally occurring mutant allele in maize (C-I) has been postulated to encode an inhibitor of anthocyanin biosynthesis which operates via effects on transcriptional activators.. Synthetic C-I constructs have been reported by Goff et al. (Maize Genetics Newsletter, 64, 6) to inhibit Bz1::luciferase activity in a transactivation assay using exogenous plasmid-borne luciferase as a heterologous reporter. No inhibition of genes native to the plant genome have been heretofore reported using dominant trans-acting inhibitor genes constructed in vitro.

**Brief Description of the Drawing**

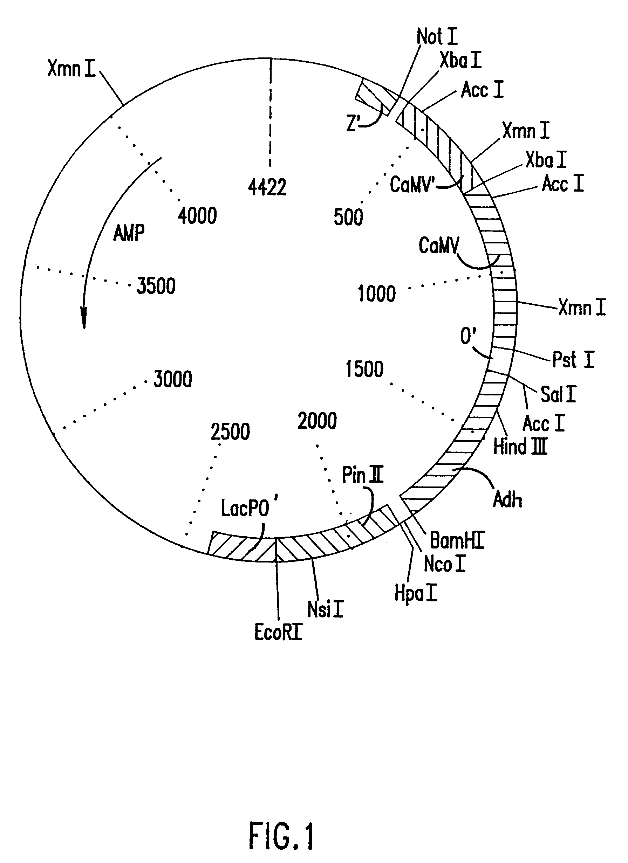

In the Figures the following symbols are used:
B BamHI
Bc BclI
Bg BglII
E EcoRI
H HindIII
X XbaI
Fig. 1 shows a plasmid map of pPHI289.

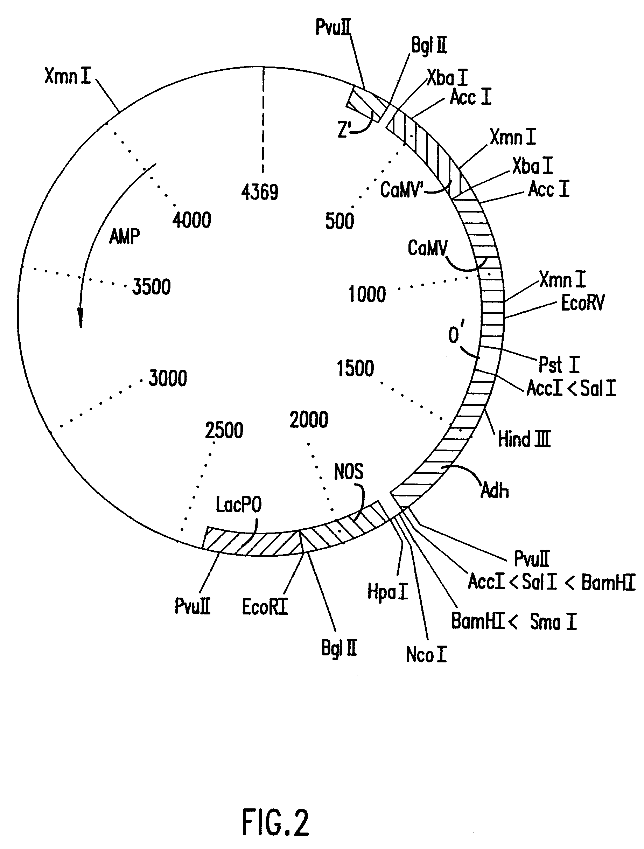

Fig. 2 shows a plasmid map of pPHI414.

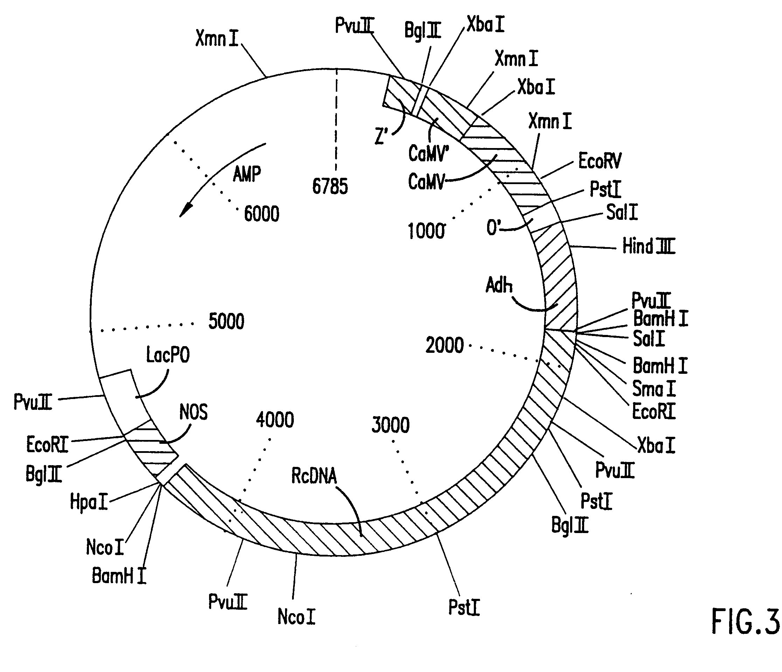

Fig. 3 shows a plasmid map of pPHI443.

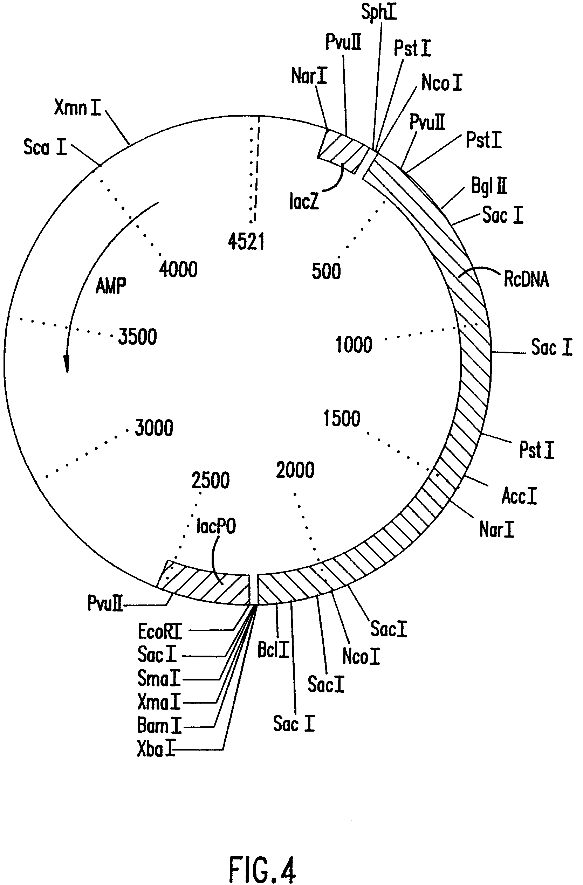

Fig. 4 shows a plasmid map of pPHI466.

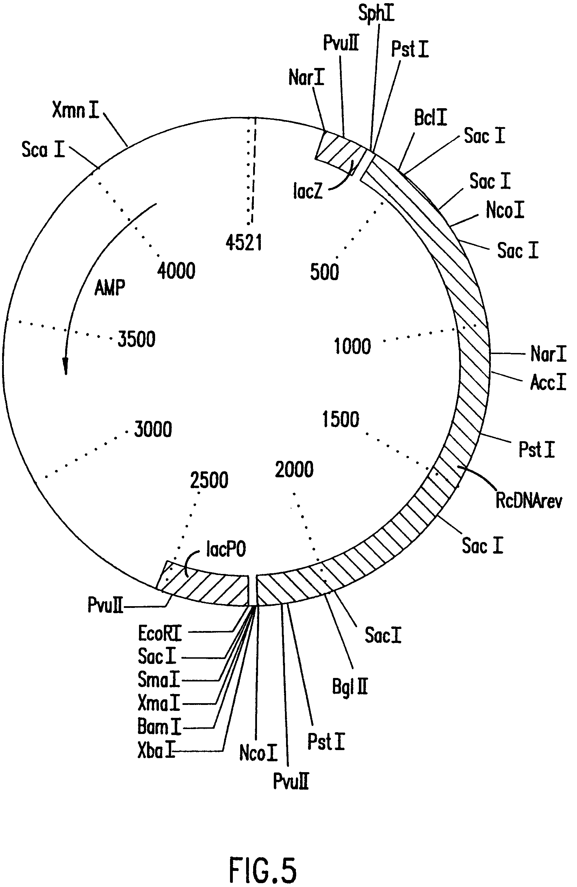

Fig. 5 shows a plasmid map of pPHI467.

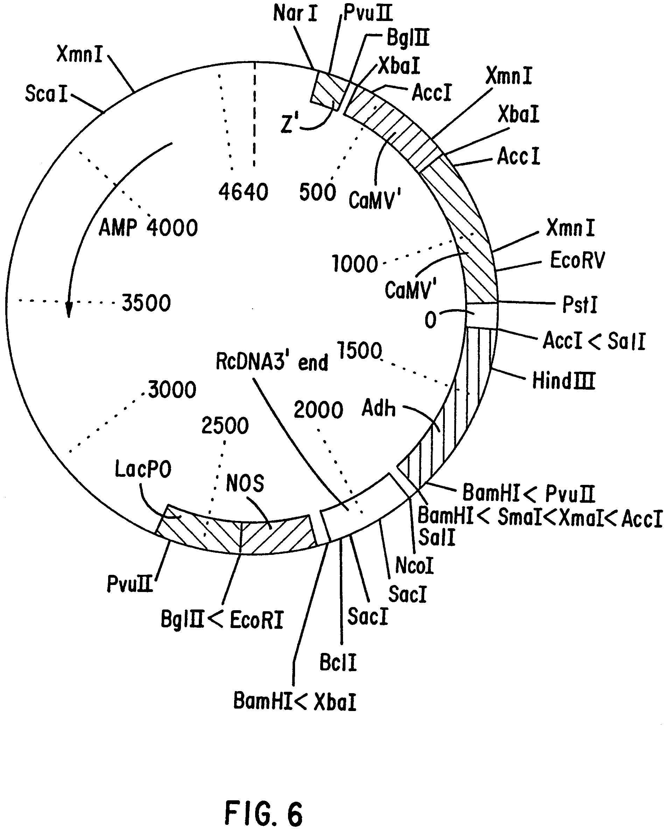

Fig. 6 shows a plasmid map of pPHI470.

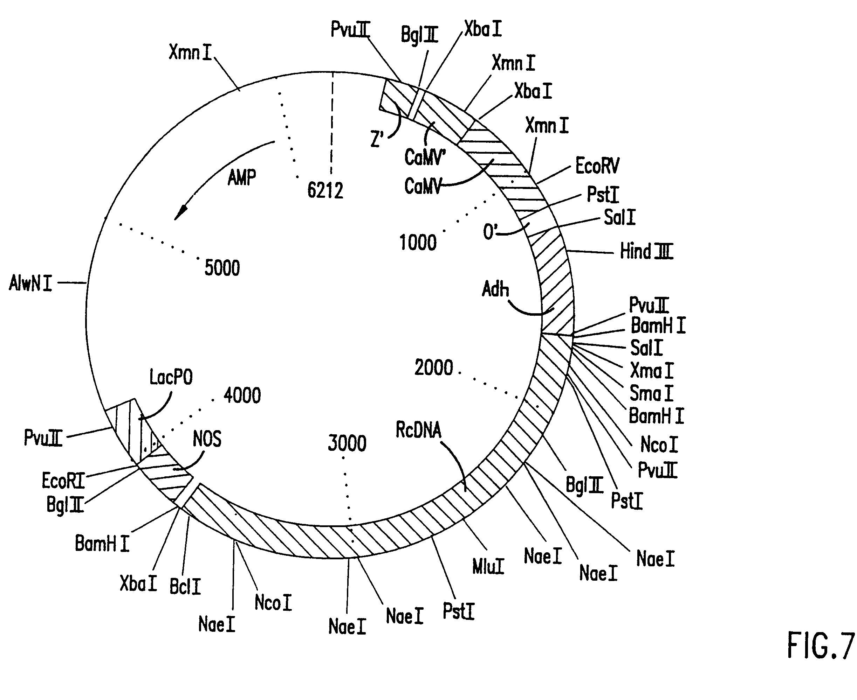

Fig. 7 shows a plasmid map of pPHI471.

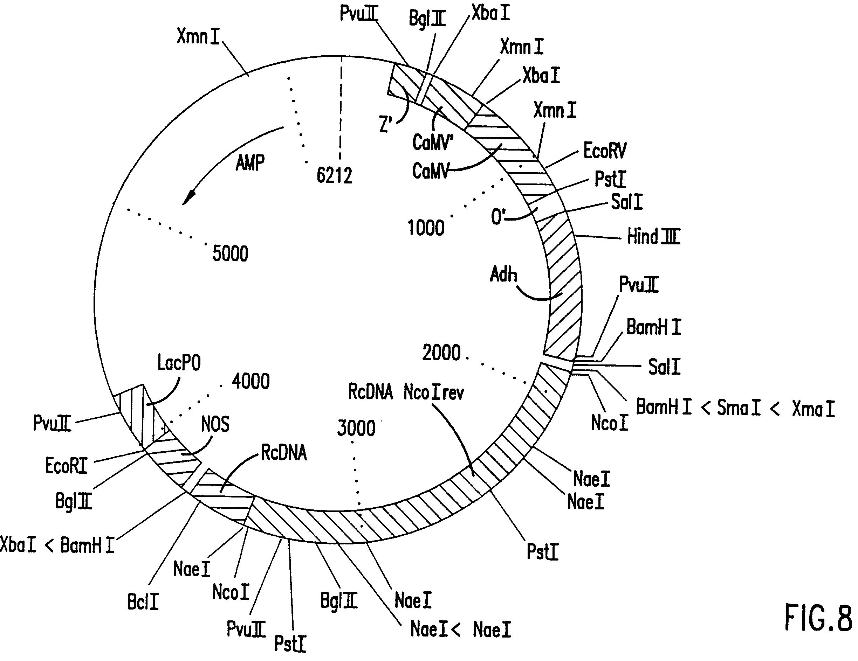

Fig. 8 shows a plasmid map of pPHI472.

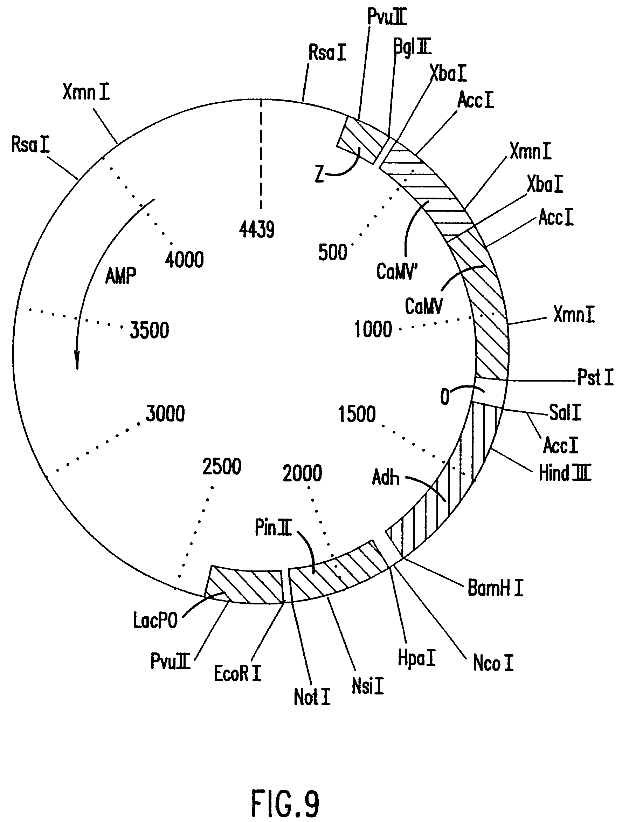

Fig. 9 shows a plasmid map of DP542.

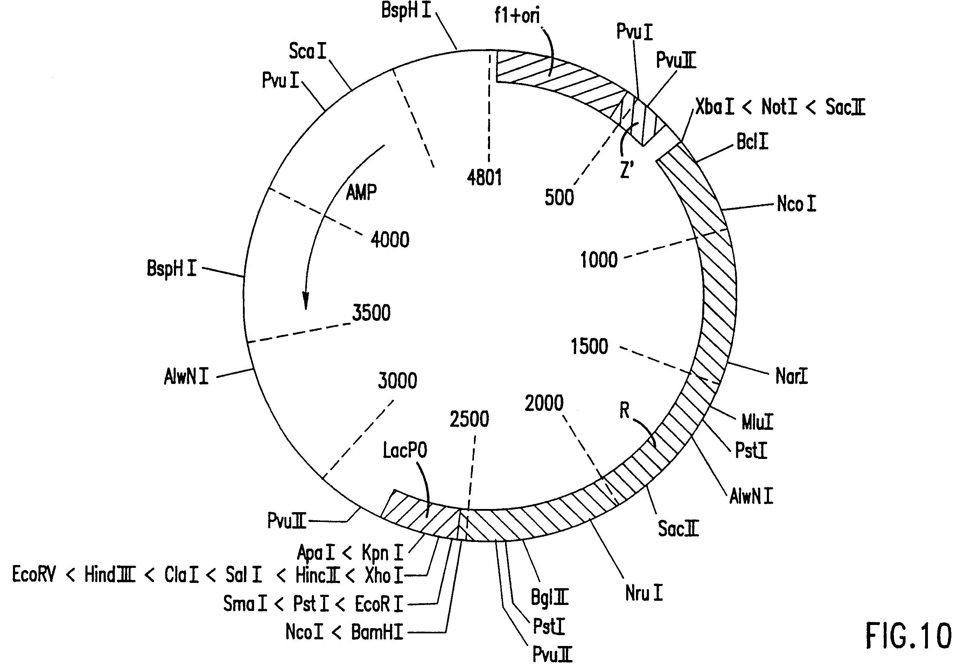

Fig. 10 shows a plasmid map of DP630.

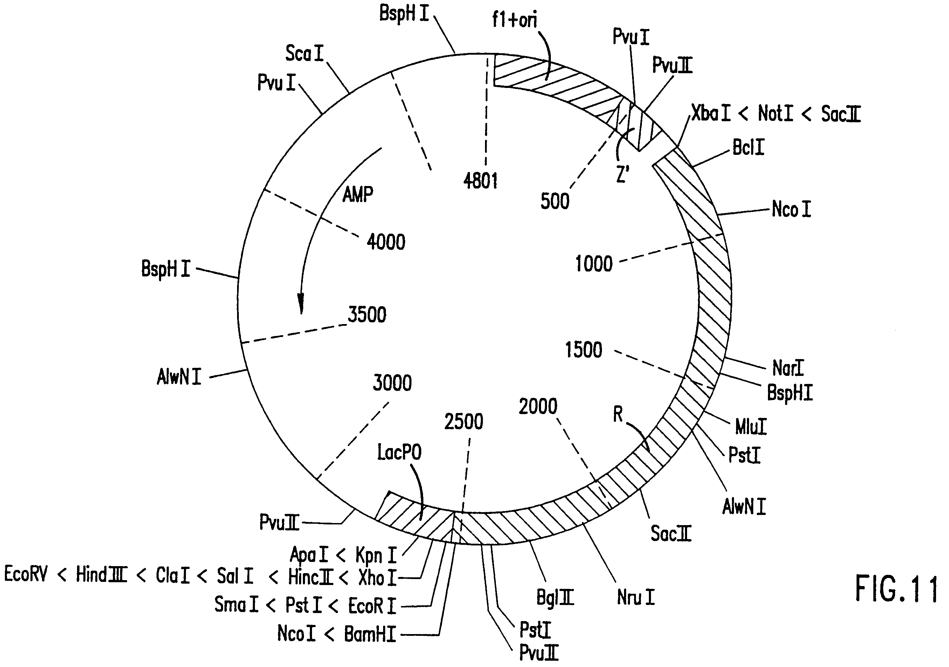

Fig. 11 shows a plasmid map of DP663.

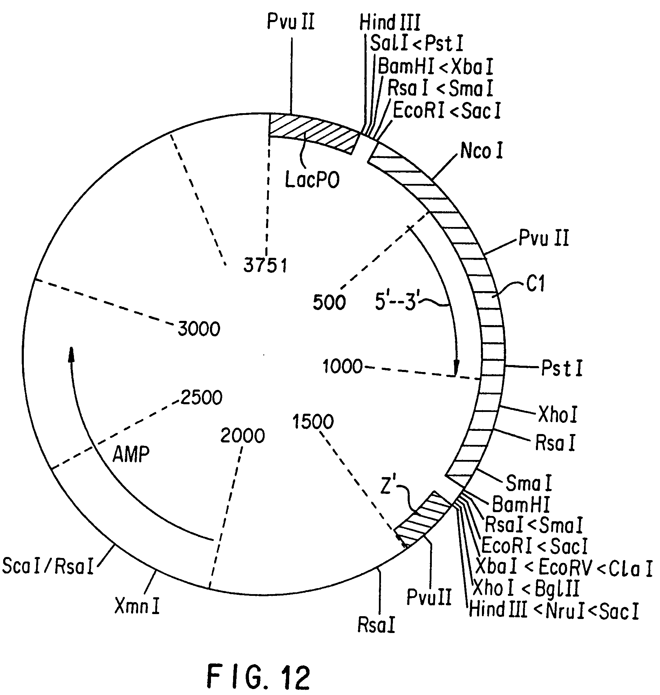

Fig. 12 shows a plasmid map of DP664.

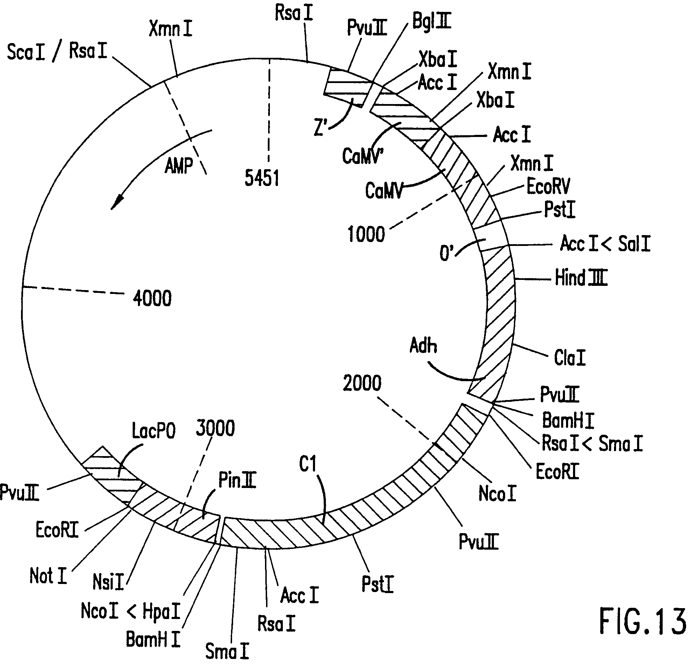

Fig. 13 shows a plasmid map of DP665.

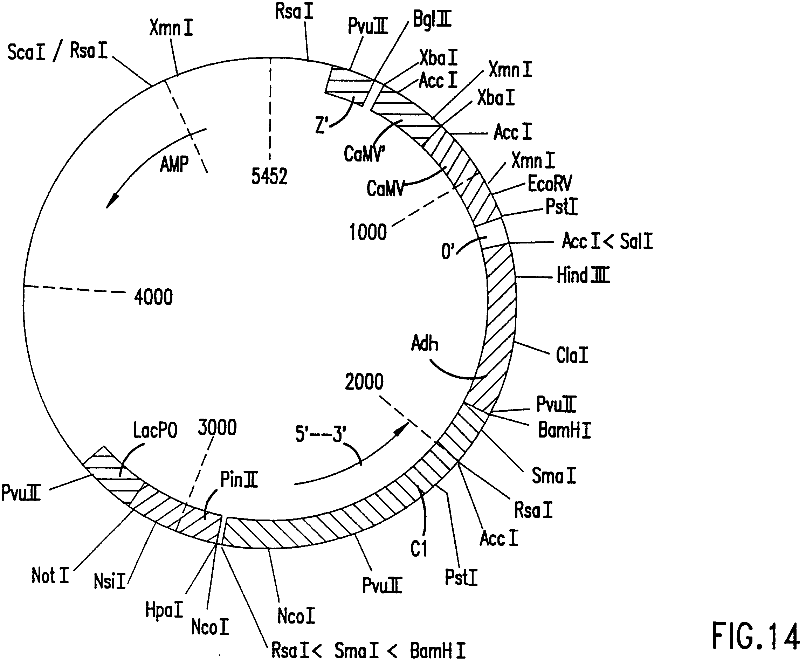

Fig. 14 shows a plasmid map of DP669.

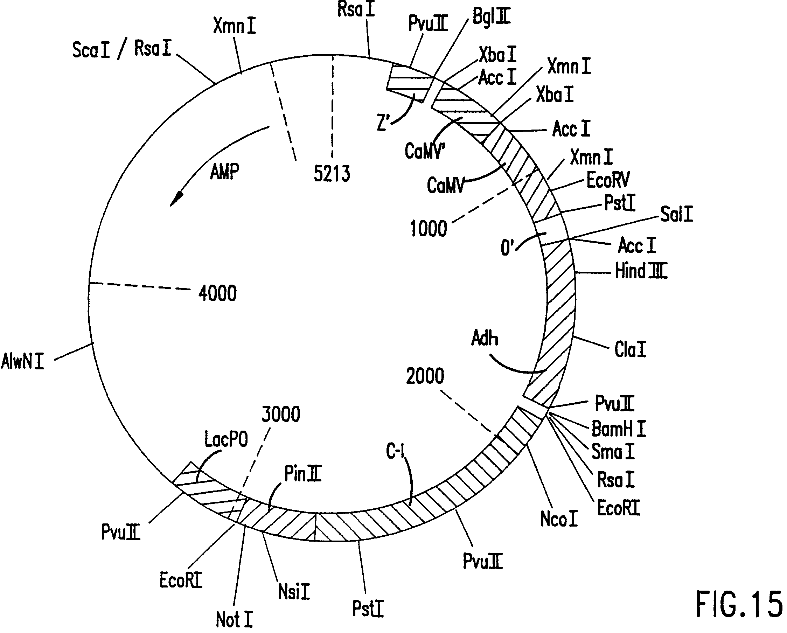

Fig. 15 shows a plasmid map of DP680.

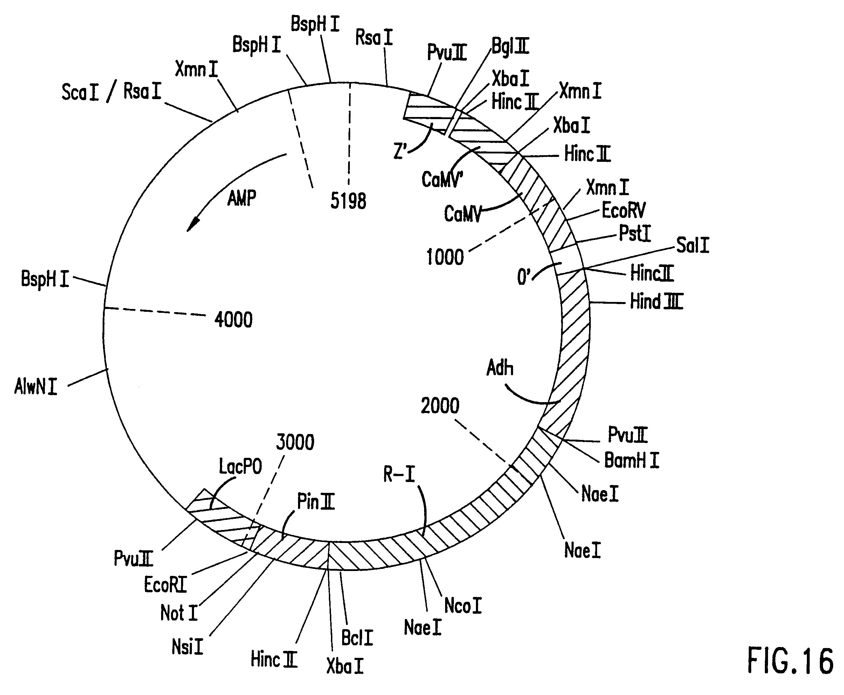

Fig. 16 shows a plasmid map of DP692.

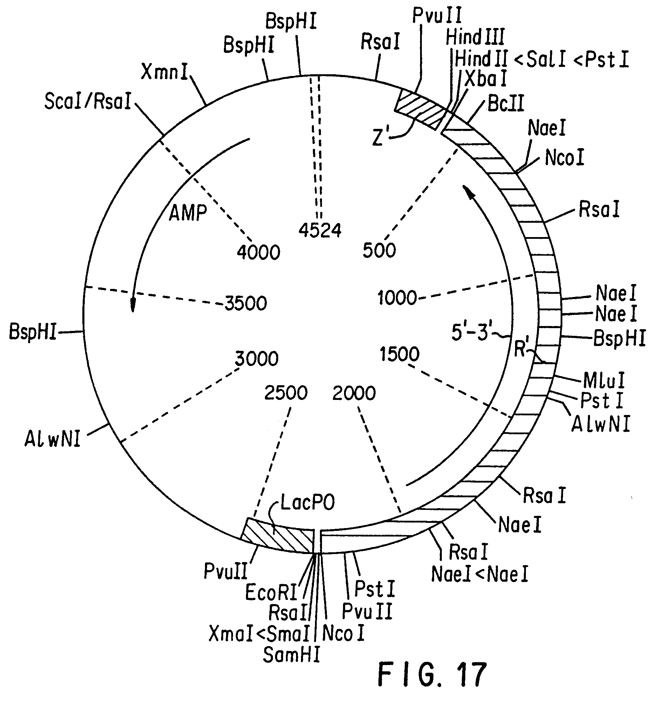

Fig. 17 shows a plasmid map of DP693.

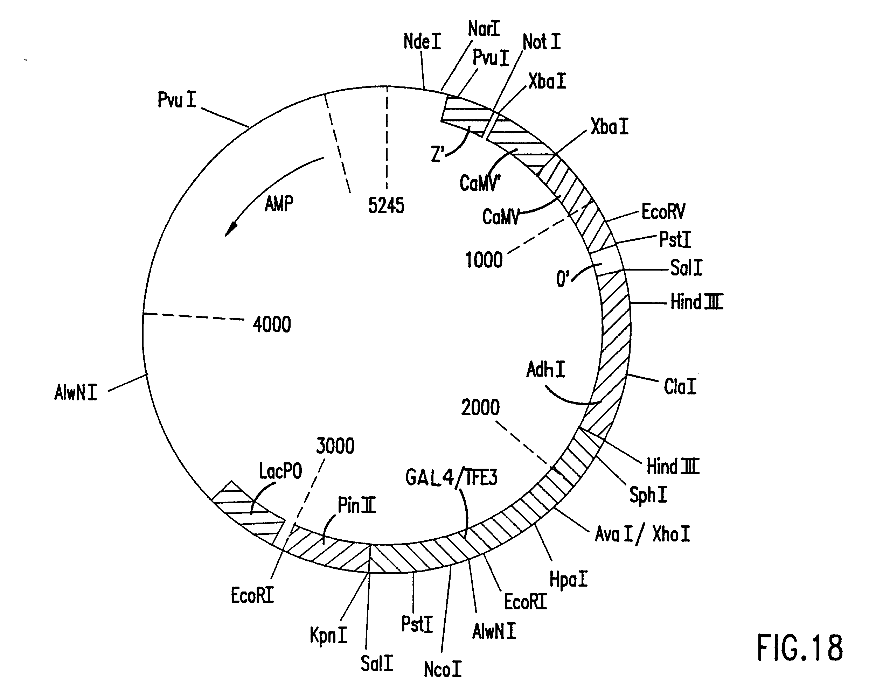

Fig. 18 shows a plasmid map of DP748.

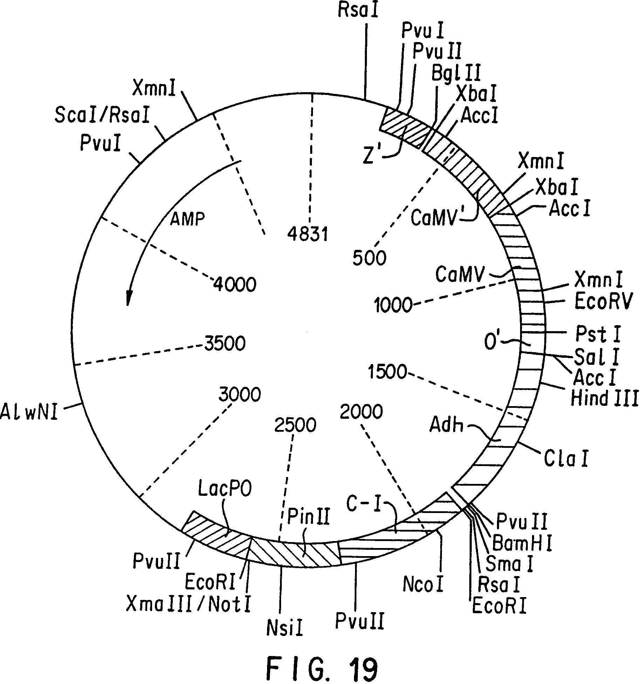

Fig. 19 shows a plasmid map of DP752.

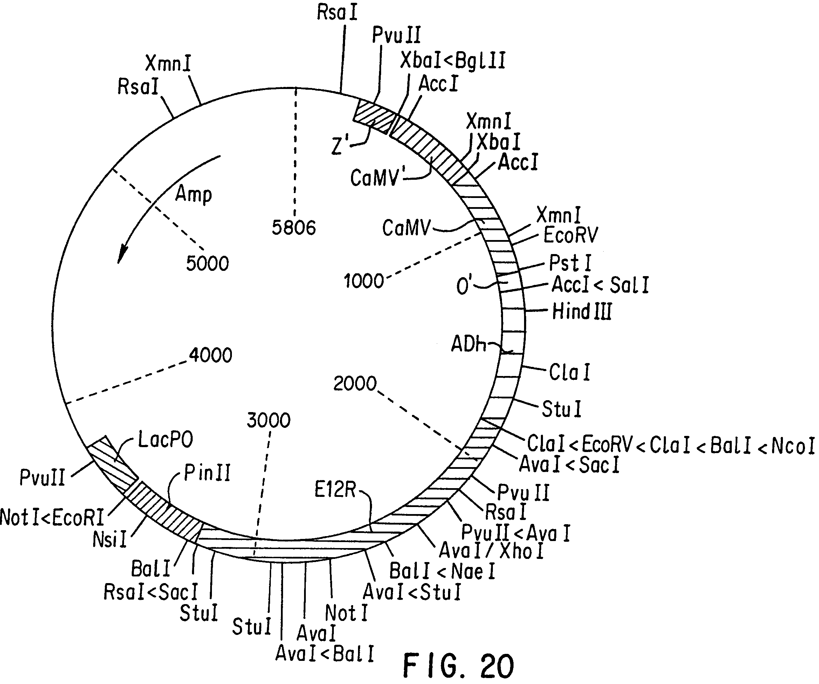

Fig. 20 shows a plasmid map of DP791.

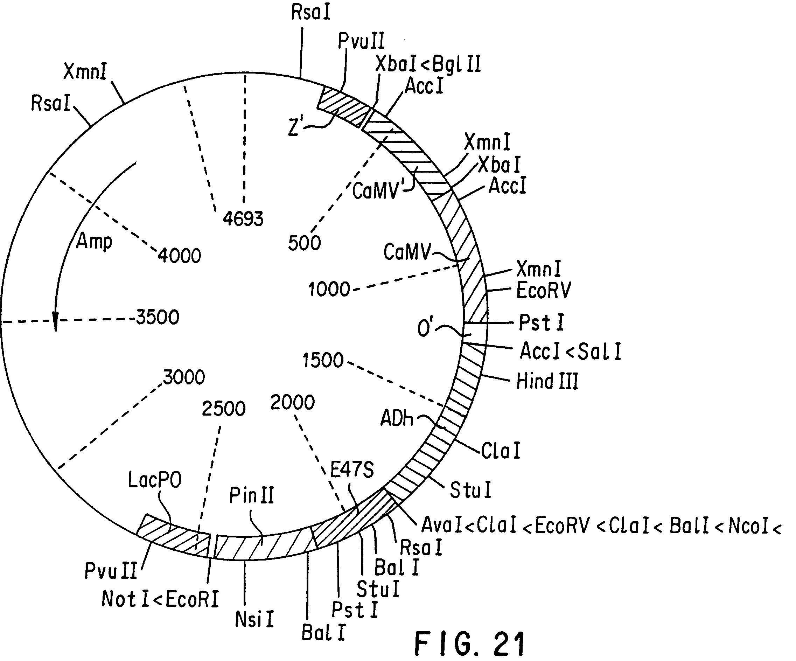

Fig. 21 shows a plasmid map of DP792.

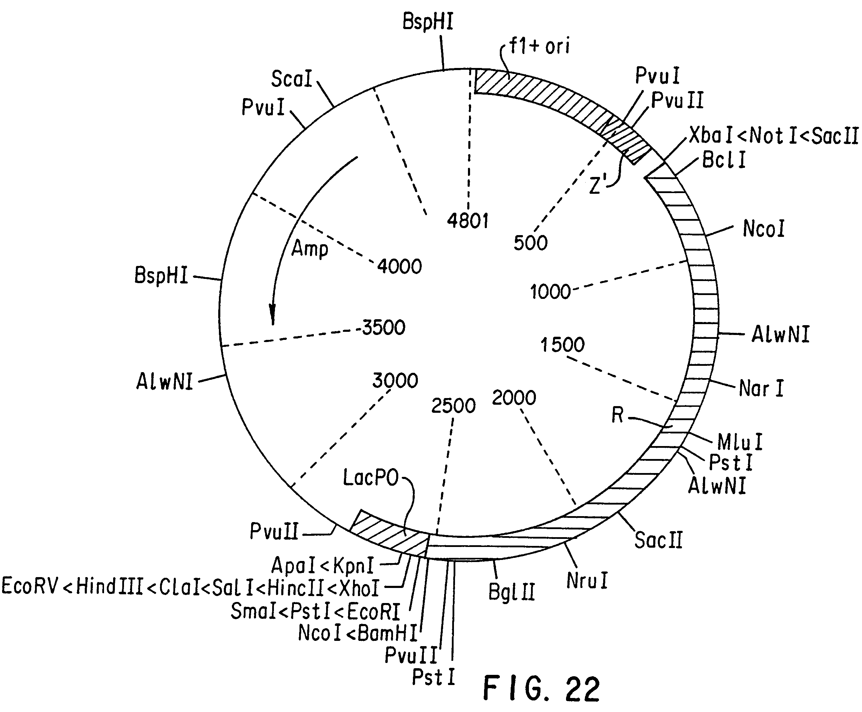

Fig. 22 shows a plasmid map of DP1202.

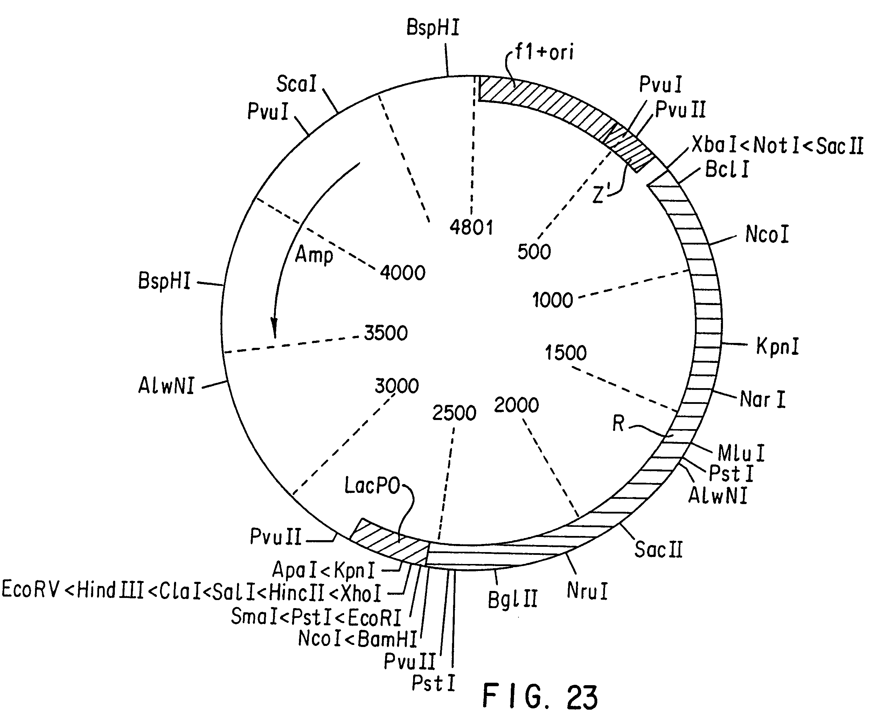

Fig. 23 shows a plasmid map of DP1206.

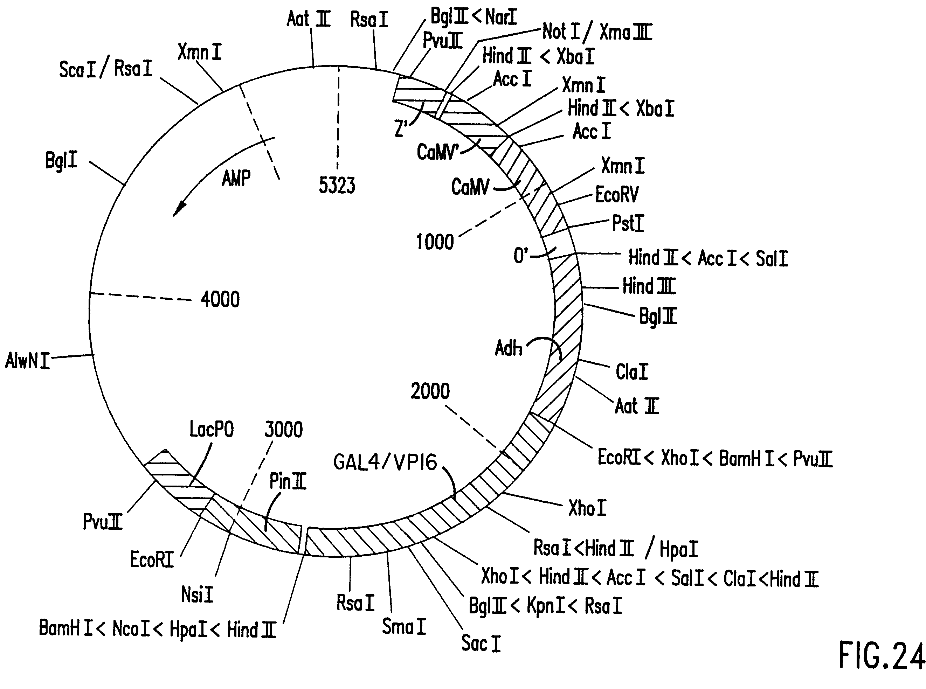

Fig. 24 shows a plasmid map of DP1209.

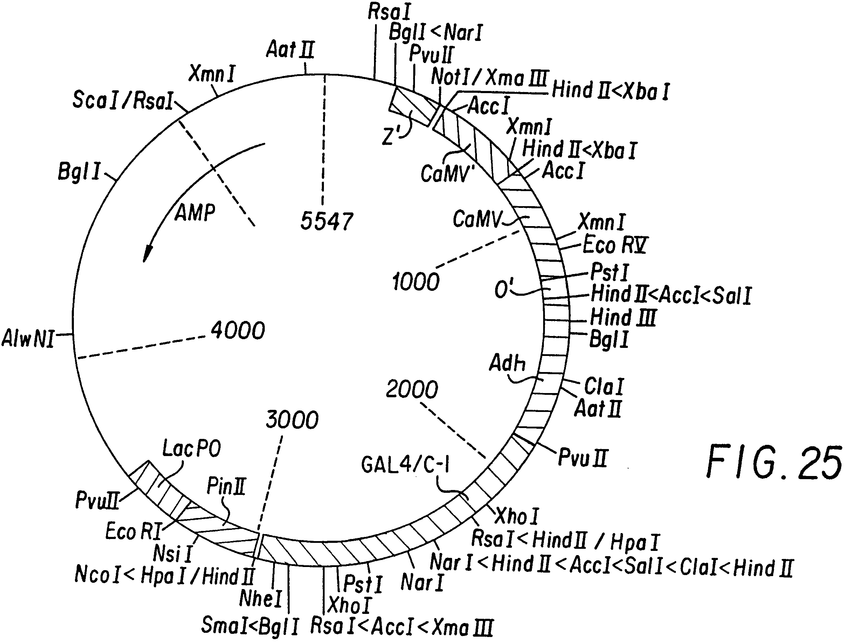

Fig. 25 shows a plasmid map of DP1211.

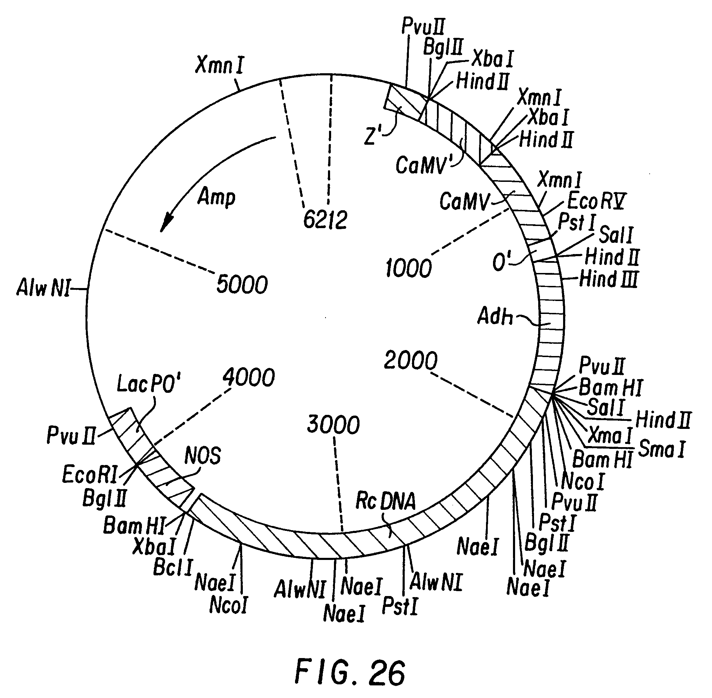

Fig. 26 shows a plasmid map of DP1216.

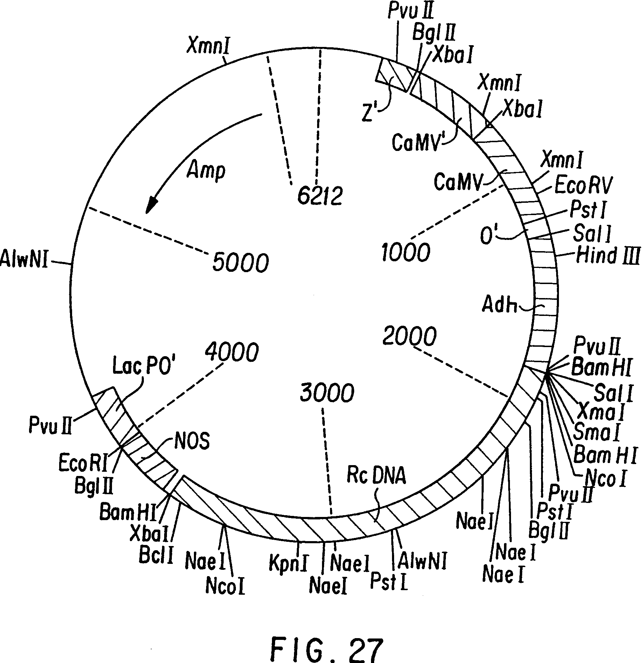

Fig. 27 shows a plasmid map of DP1217.
Fig. 28 shows two graphs illustrating the results of Examples 29 and 30.
Fig. 29 shows two graphs illustrating the results of Examples 29-32.
Fig. 30 shows a single graph illustrating the results of Examples 33 and 34.
Fig. 31 shows a single bar graph illustrating the results of Examples 35-37.

As used herein, the plasmid designation prefixes "pPHI" and "DP" are interchangeable, i.e., pPHI443 can also be referred to as DP443, and DP1211 can also be referred to as pPHI1211.

**Disclosure of the Invention**

Gene expression can be controlled by regulatory proteins that bind to specific sites on DNA. These proteins are called activators or repressors depending on whether they increase or decrease transcription; some proteins can perform both functions. While not intending to be limited by theory, the activators appear to function by binding to specific recognition sites on a DNA sequence. This site or sites y be close to the gene in terms of sequence, or, because looping of DNA permits sequence-distant regions to be physically close to each other, it may be a considerable distance along the genome from it. Binding of one or more transcriptional activators factors enables the formation of an active transcription complex comprised of RNA polymerase and its associated proteins. Once this complex forms, transcription is initiated.

It has now been discovered that genes which encode transcriptional activators can be altered or mutated in vitro so that they encode proteins that will still bind to one or more components of the transcription complex in such a way that they will competitively inhibit expression of the target gene or genes in plants. This inhibitory activity extends to the entire set of genes subject to coordinated regulation by the selected transcriptional activator. The activity of these inhibitors in plants has now been established using endogenous anthocyanin biosynthetic genes in maize. On the basis of these results, it will be appreciated that this invention can be implemented in any monocot or dicot species, including, without limitation, maize, sorghum, wheat, triticale, barley, oats, rice, soybean, alfalfa, tobacco, canola, sunflower and tomato.

Accordingly, the present invention provides a method of inhibiting expression of one or more genes in the genome of a plant, comprising the step of introducing into the environment of the plant gene a protein having substantially the amino acid sequence of the transcriptional activator of the gene, but having an amino acid sequence which differs from the amino acid sequence of the transcriptional activator of the gene by at least one amino acid, whereby the protein is unable to activate transcription of the gene. In a preferred embodiment, the protein is introduced into the environment of the plant gene by introducing into the plant cell a DNA sequence which codes for the protein.

The gene inactivation system of this invention offers the further advantage of Mendelian dominance, which makes it useful in the development and production of hybrid crops.

**Modification of Transcriptional Activators**

Most transcriptional activators have two distinct domains, the recognition domain, by which the activator recognizes and binds to DNA, and the activation domain, which somehow enhances the binding or activity of one or more components of the transcriptional machinery, such as RNA polymerase.

As described above, certain naturally occurring mutants which produce repressor proteins which bind to DNA or one or more components of the transcription complex but fail to activate transcription have been identified. Analysis of these proteins in some instances show certain amino acid substitutions which decrease the amount of negative charge in or near the activation domain of the molecule. Accordingly, activation domains can be functionally identified using trans-activation assays, such as GAL4 fusions in yeast. From that information and/or the amino acid sequence of the protein, specific alterations which result in modifications in the activation domain can be designed. From these structural modifications and the corresponding changes in gene sequence, genes can be synthesized which will produce the modified transcriptional activator protein when introduced into the cell. In some instances, structural information on the transcriptional activator or a structurally related transcriptional activator is known, and the modification can be performed on the basis of that information. Thus, this invention can be practiced by a method which includes the steps of (a) isolating the transcriptional activator protein for the gene, the protein having a recognition domain and an activation domain; (b) altering the amino acid sequence of the protein to reduce its ability to activate transcription of the gene; (c) providing a DNA sequence which codes for the altered protein; and (d) introducing the DNA sequence into the plant cell, whereby the altered protein is expressed to block transcription of the gene.

While not intending to be limited by theory, there are a number of mechanisms by which an altered transcriptional activator can operate to block transcription. By binding to DNA without activating transcription an altered protein with a normal recognition domain can compete with the genuine transcriptional activator and occupy its intended binding site. A second mechanism by which inhibitors of this invention can operate is by binding to the native transcriptional activator to form an inactive dimer or heterodimer. A preferred protein for these purposes can be constructed simply by deleting the activation domain of the native transcriptional activator, and will consist essentially of the recognition domain from a plant gene transcriptional activator protein having both a recognition domain and an activation domain.

A third method is by binding to an adjacent site on the DNA molecule so that protein-protein interactions with the genuine transcriptional activator prevent the activator from binding with the transcription complex. A fourth mechanism is by binding to the gene at or near the transcription start site, blocking the interaction of the transcription complex with the gene. These latter two mechanisms require a protein having a recognition domain which is different from the native transcriptional activator. This can be accomplished by addition, deletion, or replacement of one or more codons in the gene which codes for the native transcriptional activator, resulting in a gene product which has at least one added, missing, or different amino acid in its sequence as compared to the native transcriptional activator.

The methods of the present invention can be applied to regulatory proteins regardless of their method of recognition and binding, as described above. A fifth mechanism requires overexpression of an acidic activation domain which titrates out the target of the transcriptional activator. This phenomenon has been observed in yeast and mammalian cells and is referred to as "squelching." See, e.g., Ptashne, Nature, 335:683-689 (1988); and Ptashne et al., Nature, 346:329-331 (1990). This mechanism has now been implemented in plant cells as shown in Examples 35-37, below.

**Industrial Applicability**

The following examples are intended to be illustrative of the present invention without being limitative thereof.

Example 1

pPHI289

The plasmid pPHI289 is an empty expression cassette for use in monocots, derived from a pUC18 plasmid containing an enhanced 35S promoter spanning nucleotides - 421 to +2 of Cauliflower Mosaic Virus with the region from - 421 to - 90 duplicated in tandem, a 79 bp HindIII SalI fragment from pJII101 spanning the 5′ leader sequence of Tobacco Mosaic Virus as described by Gallie et al., Nucleic Acids Research, 15:3257 (1987), a 579 bp fragment spanning the first intron from maize Adh1-S as described by Dennis et al., Nucleic Acids Research, 12:3983 (1984), and a fragment spanning the polyadenylation site from the potato protease inhibitor II gene as described by An et al., Plant Cell, 1:115-122 (1989). Genes coding for the modified proteins of this invention can be inserted into this vector for expression either in bacteria or in plants.

Example 2

pPHI414

The plasmid pPHI414 is an empty expression cassette for use in monocots, derived from a pUC18 plasmid containing an enhanced 35S promoter spanning nucleotides - 421 to +2 of Cauliflower Mosaic Virus with the region from - 421 to - 90 duplicated in tandem, a 79 bp HindIII SalI fragment from pJII101 spanning the 5′ leader sequence of Tobacco Mosaic Virus, a 579 bp fragment spanning the first intron from maize Adh1-S, and a 281 bp fragment spanning the polyadenylation site from the nopaline synthase gene in pTiT37 as described by Bevan et al., Nucleic Acids Research, 11:369 (1983). Genes coding for the modified proteins of this invention can be inserted into this vector for expression either in bacteria or in plants.

Example 3

pPHI443

The plasmid pPHI443 is a pUC18 plasmid containing an enhanced 35S promoter spanning nucleotides - 421 to +2 of Cauliflower Mosaic Virus with the region from - 421 to - 90 duplicated in tandem, a 79 bp HindIII SalI fragment from pJII101 spanning the 5′ leader sequence of Tobacco Mosaic Virus, a 579 bp fragment spanning the first intron from maize Adh1-S, a 2415 bp Xba1 fragment spanning the R cDNA, and a 281 bp fragment spanning the polyadenylation site from the nopaline synthase gene in pTiT37. This construct was used in Ludwig et al., Science, 247:449-450 (1990).

Example 4

pPHI466

The R cDNA of pPHI443 was used as a template for PCR. The oligonucleotides used introduced a NcoI side at the 5′ end of the R gene. The resulting sequence was end filled and inserted into the HindII site of pUC18.

Example 5

pPHI467

This vector was prepared identically to pPHI466 except that the R gene was cloned in the opposite orientation.

Example 6

pPHI470

A 270 bp NcoI/SmaI fragment of the R gene from DP466 was cloned into the NcoI/HpaI sites of DP414.

Example 7

pPHI471

The 1.5 kb 5′ portion of the R gene from DP466 contained in an NcoI fragment was cloned in the NcoI site of DP470. The construct resulting in reconstitution of the R gene (sense version) was isolated and used in the experiments of Examples 28-37.

Example 8

pPHI472

This vector was prepared identically to pPHI471 except that the 1.5 kb fragment was cloned in the antisense orientation.

Example 9

DP542

The plasmid pPHI542 is an empty expression cassette for use in monocots. It is identical to pPHI289 with the exception of a NotI restriction site added downstream of the plant transcription unit. Genes coding for the modified proteins of this invention can be inserted into this vector for expression either in bacteria or in plants.

Example 10

DP630

The vector DP471 was digested with BamHI and XbaI to yield a 1.8 kb fragment containing the entire R gene. This was cloned into the BamHI/XbaI sites of pBluescript KS+ (Stratagene). The resulting vector was isolated and designated DP630.

Example 11

DP663

DP630 was subjected to site directed mutagenesis to change the G residue to an A at bp 1066 of the R gene. This change introduced a BspHI site and a change of amino acid 356 of the gene product from alanine to threonine.

## Example 12

### DP664

pcLC28.2 was digested with EcoRI and NheI and the resulting 1.0 kb fragment containing the entire C1 gene was cloned into vector pIC20H [Marsh et al., Gene, 32:481-485 (1984)] to produce DP664.

## Example 13

### DP665

DP664 was digested with BamHI to yield a 1.029 kb C1 fragment which was cloned into DP542 which had been digested with BamHI. The C1 was in the sense orientation relative to the promoter and termination sequences. This construct was used in the experiments of Examples 28-37.

## Example 14

### DP669

This vector was prepared identically to DP665 except that the 1.029 kb C1 fragment was cloned in the antisense orientation. This construct was used in the experiment of Example 32.

## Example 15

### DP680

DP664 was digested with XhoI and the ends were filled in using the Klenow fragment of DNA polymerarase. The resulting mixture was then digested with BamHI and the resulting 801 bp fragment of the C1 gene was cloned into DP542 which had been digested with BamHI and BalI. The resulting altered C1 gene product terminated at amino acid 258.

## Example 16

### DP692

DP693 of the following example was digested with BspHI and HincII to produce a 779 bp fragment containing the 3' portion of the R gene (R-I). This was cloned into the NcoI/HpaI sites of DP542 to produce DP692. This construct was used in the experiment of Example 29.

## Example 17

### DP693

DP663 was digested with BamHI and XbaI. The resulting 1.8 kb R gene fragment was cloned into pUC18 at the BamHI/XbaI sites.

## Example 18

### DP748

The GAL4Λ3-Δ2 plasmid (Beckmann et al. Genes and Development, 4:167-179 (1990))containing an 800 bp GAL4/TFE3 fusion gene was digested with BglII/SmaI. The resulting fragment was cloned into the BamHI/SmaI sites of DP289 to produce DP748. This construct was used in the experiment of Example 37.

## Example 19

### DP752

DP680 was digested with XmaIII and the ends were filled in using the Klenow fragment of DNA polymerarase. The resulting mixture was then digested with BamHI and the resulting fragment was cloned into the BamHI/BalI sites of DP542 to produce DP752. The resulting C1 gene product terminated at amino acid 140. This construct was used in the experiment of Example 31.

## Example 20

### DP791

E12R plasmid DNA as described by Murre et al., Cell, 58:537-544 (1989) was digested with NcoI and EcoRI and the ends were filled in using the Klenow fragment of DNA polymerarase. The resulting 1.3 kb fragment was cloned into the NcoI/HpaI sites of DP542 to produce DP791. Results of using this gene in the experiment of Example 33 are illustrated in the Figures.

## Example 21

### DP792

E47S plasmid DNA as described by Murre et al., Cell, 58:537-544 (1989) was digested with NcoI and EcoRI and the ends were filled in using the Rlenow fragment of DNA polymerarase. The resulting 400 bp fragment was cloned into the NcoI/HpaI sites of DP542 to produce DP792. Results of using this gene in the experiment of Example 34 are illustrated in the Figures.

## Example 22

### DP1202

DP630 was subjected to site-directed mutagenesis to change the sequence between bp 1264 and bp 1270 of the R gene from CGAAAGC to GGTACCG. This change introduces a KpnI site and changes the amino acid sequence from Arg-Lys-Arg in the basic DNA binding region of the native R gene to Gly-Trp-Gly in the modified gene product.

## Example 23

### DP1206

DP630 was subjected to site-directed mutagenesis to change the sequence between bp 1265and bp 1272 of the R gene from GAAAGCGA to AGAAACTG. This change introduces a AlwNI site and changes the amino acid sequence from Arg-Lys-Arg in the basic DNA binding region of the native R gene to Gln-Lys-Leu in the modified gene product.

## Example 24

### DP1209

A plasmid containing a 1 kb GAL4/VP16 fusion gene as described by Sadowski et al., Nature, 335:563-564 (1988) was digested with BamHI. The resulting fragment was cloned into the BamHI sites of DP289 to produce DP1209. This construct was used in the experiment of Example 36.

## Example 25

### DP1211

A plasmid containing a 1 kb GAL4/C1 fusion gene was digested with BamHI. The resulting fragment was cloned into the BamHI sites of DP289 to produce DP1211. This construct was used in the experiment of Example 35.

## Example 26

### DP1216

DP1202 was digested with MluI and BclI. The resulting 858 bp fragment was cloned into the MluI/BclI sites of DP471 to reconstruct the R gene with a basic region mutation. The resulting vector was designated DP1216.

## Example 27

### DP1217

DP1206 was digested with MluI and BclI. The resulting 858 bp fragment was cloned into the MluI/BclI sites of DP471 to reconstruct the R gene with a basic region mutation. The resulting vector was designated DP1217.

## Example 28

### Inhibition of Gene Expression

Sample preparation

Plasmid DNA was purified from E. coli by alkaline lysis and PEG precipitation. Tungsten particles (1.2 microns avg. dia.) from General Electric Co. were washed in ethanol 4 to 5 times and 4.375 mg. aliquots were evaporated to dryness in microfuge tubes. DNA preparations containing 10 μg total DNA in 10 μL TE buffer (10 mM Tris-HCl, 1 mM EDTA, pH 8.0) comprising the experimental constructs of the foregoing examples were added to the particles. Each of the DNA preparations contained DP665 (0.5 μg except as noted below) and DP471. All preparations contained the same copy number of 35S promoter/enhancer sequences to avoid differential titration of transcription factors which bind to the 35S constructs. Where additional 35S sequences were needed, they were provided by the "blank" 35S::GUS construct pPHI459 developed by Ploneer Hi-Bred International, Inc. and described in Ludwig et al., Science,247:449-450 (1990). All plasmids were approximately the same size. After the DNA was added to the particles, the particles were resuspended by brief sonication and then 25 microliters of 2.5 M CaCl$_2$ and 10 microliters of 0.1 M spermine were added to the preparation with additional brief sonication. After the particles settled, 30 μL of the supernatant liquid was removed.

Tissue Preparation

Maize embryogenic suspension cells 3-44-6E1 derived from a maize hybrid typical of field corn (genotype c r-r B-b pl) were grown in medium containing MS salts and vitamins, 0.1 g/L myo-inositol, 2 mg/L 2,4-D, and 30 g/L sucrose. Prior to sampling, the suspensions were sieved through a screen, weighed, and resuspended at a density of 25 mg./mL in the same medium to which 3% PEG (w/v) had been added. Cells were incubated in that medium overnight under normal growing conditions. The next day, 1 mL aliquots of the cells were placed onto 2 Whatman 617 filter papers prewetted with 1 mL modified MS medium in 6 x 2 cm petri dishes.

Each sample of cells was bombarded once with a 1 μL aliquot of particles containing the experimental mixture. Three samples of cells were bombarded separately for each DNA preparation to provide 3 reps per treatment.

After bombardment, 1 mL modified MS medium was added to each sample and the samples were incubated in darkness at 3°C for 2-3 days. Red cells were counted for all samples within a 4-6 hour period. Results were summed over all three samples per preparation to minimize bombardment-to-bombardment variations.

Example 29

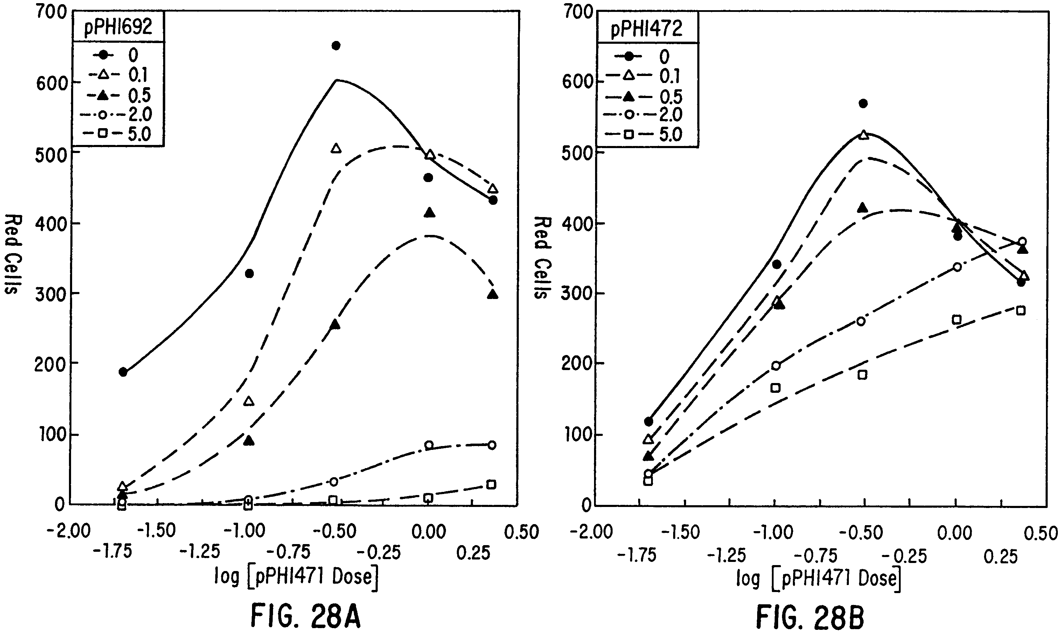

The foregoing general experimental method was used. DP471 content of the DNA preparation was varied from 0.02 μg to 5 μg, and DP692 content was varied from 0 to 5 μg. Results are illustrated in the first graph in Figure 28.

Example 30

The foregoing general experimental method was used. DP471 content of the DNA preparation was varied from 0.02 μg to 5 μg, and DP472 content was varied from 0 to 5 μg. Results are illustrated in the second graph in Figure 28.

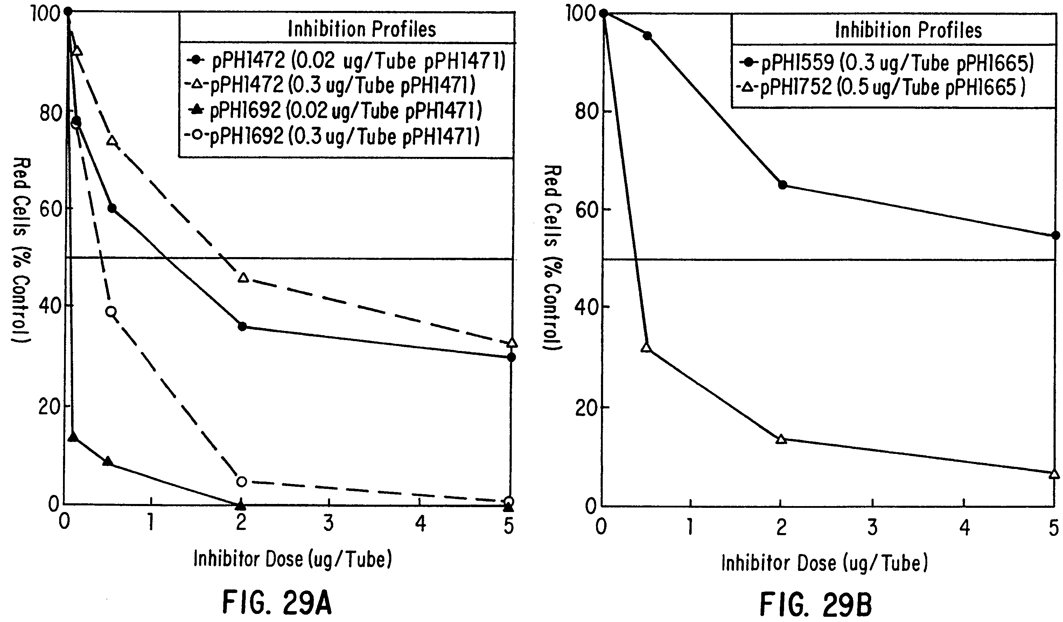

The results of these two examples show that DP472, which expresses antisense R, was 5- to 20-fold less effective than DP692, which expresses modified R (R-I). In the case of the modified R transcriptional activator (R-I), 100% inhibition of gene expression was obtained, while 100% inhibition could not be obtained with antisense R. This is also illustrated graphically in the left-hand graph of Figure 29.

Example 31

The foregoing general experimental method was used. DP471 content of the DNA preparation was fixed at 0.5 μg, and DP752 content was varied from 0 to 5 μg.

Example 32

The foregoing general experimental method was used, except that DP665 content of the DNA preparation was 0.3 μg. DP471 content of the DNA preparation was fixed at 0.5 μg. DP669 content was varied from 0 to 5 μg. Results of this and the preceding example are illustrated in the graph on the right side of Figure 29. The C-I construct (DP752) was at least 10 times more effective than the antisense construct (DP669).

Example 33

The foregoing general experimental method was used. DP471 content of the DNA preparation was fixed at 0.5 μg, and DP791 content was varied from 0 to 9 μg.

Example 34

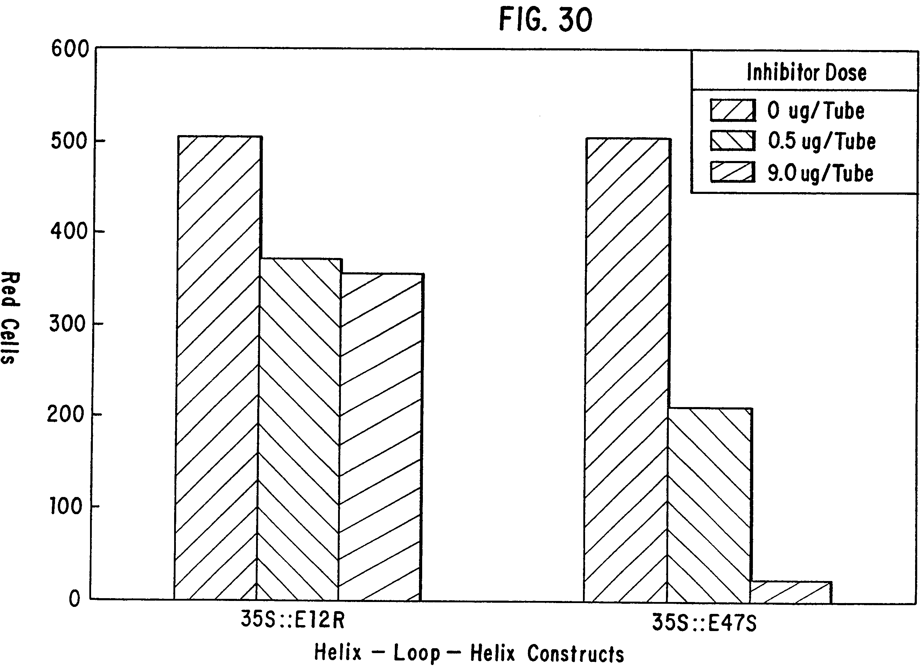

The foregoing general experimental method was used. DP471 content of the DNA preparation was fixed at 0.5 μg and DP792 content was varied from 0 to 9 μg. Results of this and the preceding experiment are illustrated in Figure 30. These results show that the construct which expresses the helix-loop-helix domain and the adjacent basic region of the human E47S protein (DP792) inhibits anthocyanin biosynthesis about as effectively as the construct which expresses R-I, DP692. The construct which expresses E12R (DP791) is not as effective an inhibitor.

Example 35

The foregoing general experimental method was used. DP471 content of the DNA preparation was fixed at 0.5 μg, and DP1211 content was varied from 0 to 9 μg.

Example 36

The foregoing general experimental method was used. DP471 content of the DNA preparation was fixed at 0.5 μg, and DP1209 content was varied from 0 to 9 μg.

Example 37

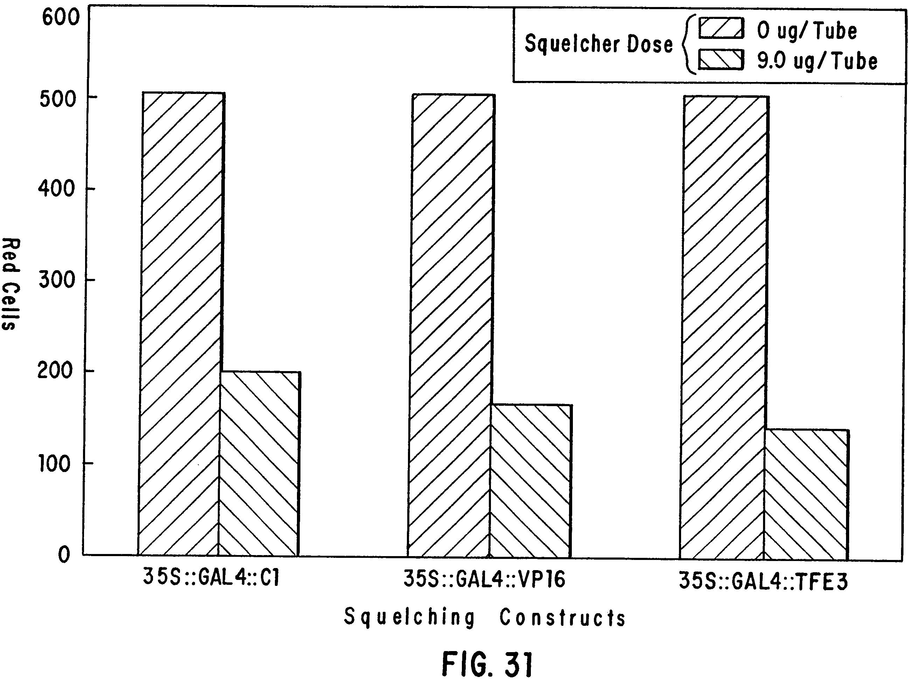

The foregoing general experimental method was used. DP471 content of the DNA preparation was fixed at 0.5 μg, and DP748 content was varied from 0 to 9 μg. Results of this and the two preceding examples are illustrated in Figure 31. These results indicate that in the presence of constructs which express strong acidic activation domains, anthocyanin biosynthesis is also inhibited. This illustrates in plants the mechanism of inhibition postulated to occur in yeast and mammalian cells and referred to as "squelching."

Although the foregoing invention has been described in some detail by way of illustration and example for purposes of clarity and understanding, it will be apparent that certain changes and modifications can be practiced by those of skill in the art within the scope of the appended claims.

Claims

1. A method of inhibiting expression of a gene in the genome of a plant, the transcription of which is activated by a transcriptional activator protein, comprising the step of introducing into the environment of the gene a protein having substantially the amino acid sequence of the transcriptional activator of the gene, but having an amino acid sequence which differs from the amino acid sequence of the transcriptional activator of the gene by at least one amino acid, whereby the protein is unable to activate transcription of the gene.

2. A method according to Claim 1, wherein the protein has a recognition domain and an activation domain, the recognition domain having substantially the amino acid sequence of the recognition domain of the transcriptional activator of the gene, and the activation domain having an amino acid sequence differing from the amino acid sequence of the activation domain of the transcriptional activator of the genes by at least one amino acid.

3. A method according to Claim 2 further comprising the step of introducing into the cell a DNA sequence which codes for the protein.

4. A method according to Claim 1, wherein the protein has a recognition domain and an activation domain, the activation domain having substantially the amino acid sequence of the activation domain of the transcriptional activator of the gene, and the recognition domain having an amino acid sequence differing from the amino acid sequence of the recognition domain of the transcriptional activator of the genes by at least one amino acid.

5. A method according to Claim 4 further comprising the step of introducing into the cell a DNA sequence which codes for the protein.

6. A method according to Claim 1 further comprising the steps of:
    (a) characterizing the structure or the sequence of the transcriptional activator protein for the gene;
    (b) altering the amino acid sequence of the protein to reduce its ability to activate transcription of the gene;
    (c) providing a DNA sequence which codes for the altered protein; and
    (d) introducing the DNA sequence into the plant cell, whereby the altered protein is expressed to block transcription of the gene.

7. A DNA sequence which codes substantially solely for a protein having substantially the amino acid sequence of a transcriptional activator of a gene in a plant genome, but having a coding sequence which differs from the sequence which codes for the transcriptional activator by at least one base pair, so that the protein has an amino acid sequence which differs from the amino acid sequence of the transcriptional activator of the gene by at least one amino acid.

8. A DNA sequence according to Claim 7 wherein the coding sequence differs from the sequence which codes for the transcriptional activator by addition or deletion of a single base pair, so that a frame shift of the coding sequence exists downstream of the addition or deletion.

9. A DNA sequence according to Claim 7 which codes substantially solely for a protein consisting essentially of (a) the recognition domain of a plant gene transcriptional activator; and (b) an activation domain which differs from the activation domain of the plant gene transcriptional activator by at least one amino acid.

10. A DNA sequence according to Claim 9 wherein the sequence which codes for the activation domain differs from the native sequence for the native activation domain by deletion of one or more codons, whereby the activation domain of the protein differs from the native activation domain by deletion of one or more amino acids.

11. A DNA sequence according to Claim 9 wherein the sequence which codes for the activation domain differs from the sequence for the native activation domain by addition of one or more codons, whereby the activation domain of the protein differs from the native activation domain by addition of one or more amino acids.

12. A DNA sequence according to Claim 9 wherein the sequence which codes for the activation domain differs from the native sequence for the native activation domain by replacement of one or more codons with codons which code for different amino acids, whereby the activation domain of the protein differs from the native activation domain by replacement of one or more amino acids.

13. A DNA sequence which has as its sole gene product the recognition domain of a plant gene transcriptional activator protein.

14. A protein consisting essentially of the recognition domain from a plant gene transcriptional activator protein.

15. An expression cassette comprising a DNA sequence according to Claim 7 operably linked to plant regulatory sequences which cause the expression of the DNA sequence in plant cells.

16. A bacterial transformation vector comprising an expression cassette according to Claim 15, operably linked to bacterial expression regulatory sequences which cause replication of the expression cassette in bacterial cells.

17. Bacterial cells containing as a foreign plasmid at least one copy of a bacterial transformation vector according to Claim 16.

18. Transformed plant cells containing as foreign DNA at least one copy of the DNA sequence of an expression cassette according to Claim 15.

19. Transformed cells according to Claim 18, further characterized in being cells of a monocot species.

20. Transformed cells according to Claim 19, further characterized in being maize, sorghum, wheat, triticale or rice cells.

21. Transformed cells according to Claim 18, further characterized in being cells of a dicot species.

22. Transformed cells according to Claim 21, further characterized in being soybean, alfalfa, tobacco, sunflower, canola or tomato cells.

23. A maize cell or tissue culture comprising cells according to claim 15.

24. A transformed maize plant, the cells of which contain as foreign DNA at least one copy of the DNA sequence of an expression cassette according to Claim 15.

FIG.1

FIG.2

FIG.3

FIG.4

FIG.5

FIG. 6

FIG.7

EP 0 475 584 A2

FIG.8

EP 0 475 584 A2

FIG.9

FIG.10

FIG.11

FIG. 12

FIG.13

FIG.14

FIG.15

FIG.16

FIG. 17

EP 0 475 584 A2

FIG.18

F I G. 19

FIG. 20

FIG. 21

FIG. 22

FIG. 23

FIG.24

FIG. 25

FIG. 26

FIG. 27

FIG. 28A

FIG. 28B

EP 0 475 584 A2

FIG. 29A

FIG. 29B

FIG. 30

Red Cells

Inhibitor Dose

⬜ 0 ug/Tube
⬜ 0.5 ug/Tube
⬜ 9.0 ug/Tube

600

500

400

300

200

100

0

35S::E12R                    35S::E47S

Helix — Loop — Helix Constructs

EP 0 475 584 A2

FIG. 31

EP 0 475 584 A2